(19) Europäisches Patentamt
European Patent Office
Office européen des brevets

(11) EP 4 768 474 A1

(12) EUROPEAN PATENT APPLICATION

(43) Date of publication:
**01.07.2026 Bulletin 2026/27**

(21) Application number: **25213183.4**

(22) Date of filing: **04.11.2025**

(51) International Patent Classification (IPC):
***C07D 311/72*** *(2006.01)* ***A61K 8/67*** *(2006.01)*

(52) Cooperative Patent Classification (CPC):
**C07D 311/72; A61K 8/678;** A61K 2800/522; Y02P 20/584

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **04.12.2024 CN 202411770621**

(71) Applicant: **Shanghai Coachchem Technology Co., Ltd.**
**Shanghai 201500 (CN)**

(72) Inventors:
- **WU, Jiang**
  **Shanghai (CN)**
- **ZHANG, Wei**
  **Shanghai (CN)**
- **GAO, Ang**
  **Shanghai (CN)**
- **HAN, Tengfei**
  **Shanghai (CN)**
- **ZHOU, Baoping**
  **Shanghai (CN)**
- **ZHANG, Die**
  **Shanghai (CN)**

(74) Representative: **Becker, Eberhard**
**Becker Kurig & Partner**
**Patentanwälte mbB**
**Bavariastraße 7**
**80336 München (DE)**

# (54) TOCOPHEROL DERIVATIVE, PREPARATION METHOD THEREFOR, AND USE THEREOF

(57) The invention provides a tocopherol derivative, preparation method therefor, and uses thereof. The tocopherol derivative is a compound with the following structure.

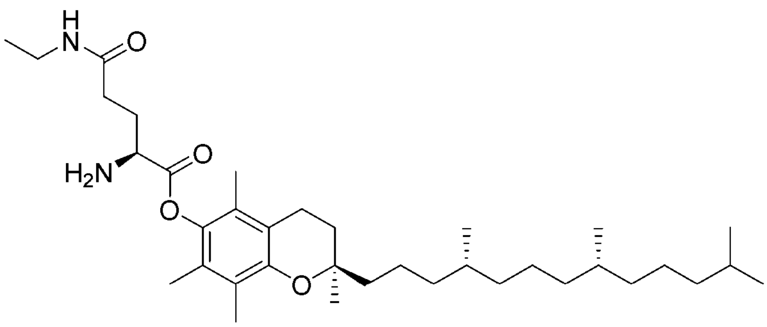

wherein R' is hydrogen or an acetyl group; the theanine and tocopherol are esterified in the presence of a catalyst and a co-catalyst to obtain the tocopherol derivative. The compound can be used as an antioxidant in cosmetics, food and health products, pet products, etc. The method has mild reaction conditions, simple operation, high yield, high synthesis efficiency, economic and environmental protection, and has good application prospects.

EP 4 768 474 A1

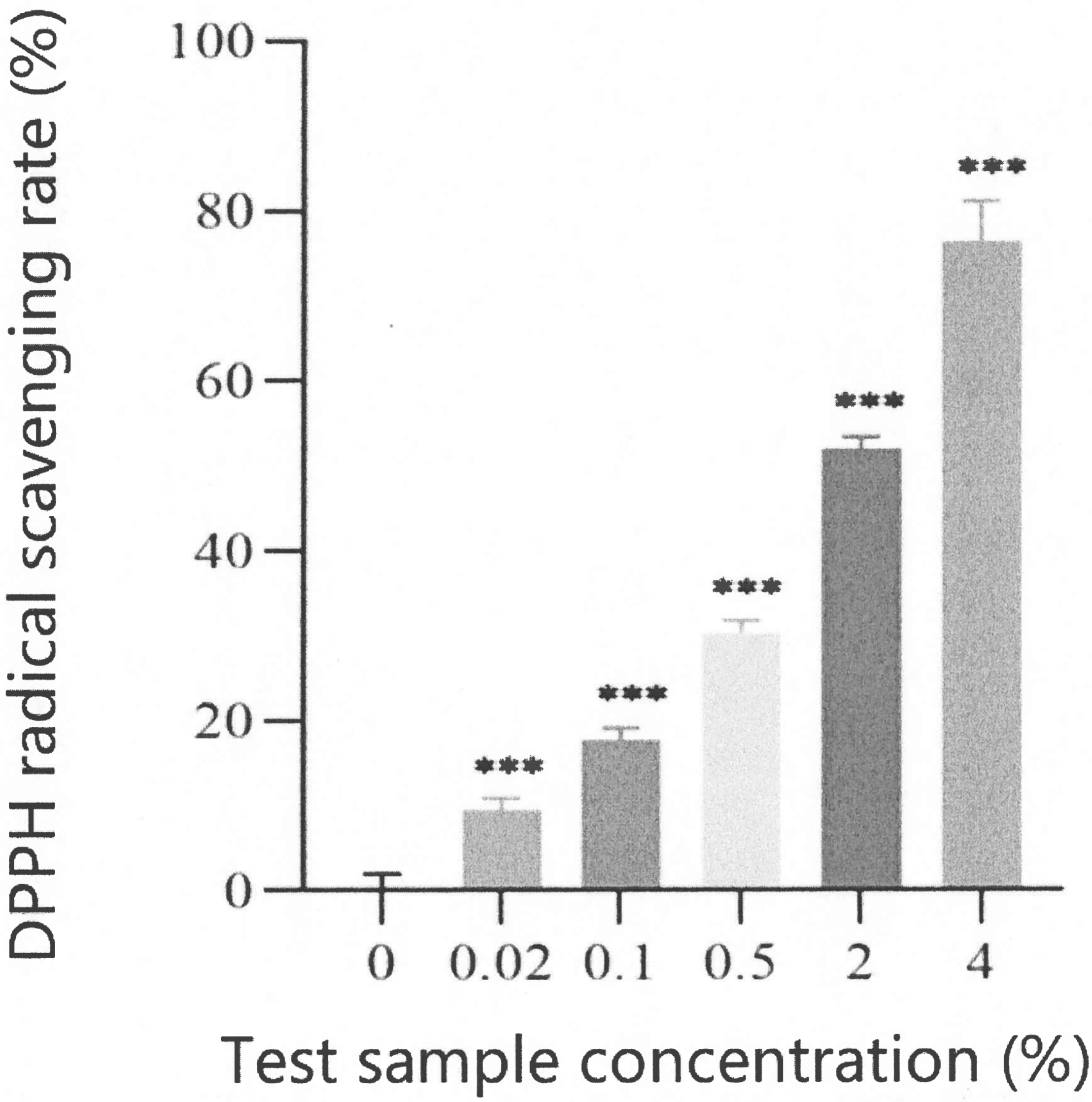
DPPH radical scavenging rate (%)
100
80
60
40
20
0
***
***
***
***
***
0
0.02
0.1
0.5
2
4
Test sample concentration (%)

FIG. 3

## Description

### TECHNICAL FIELD

The present invention relates to a novel chemical compound and a method of preparation thereof, and more specifically, to a tocopherol derivative, its catalytic synthesis method, and use thereof.

### BACKGROUD

Tocopherol and theanine have attracted considerable attention in the industry due to their antioxidant functions. It is expected that combining the two may yield a product with synergistic or enhanced effects.

However, according to existing related technologies concerning the reaction conditions of phenols and acids, attempts have been made to condense theanine and tocopherol in tetrahydrofuran to obtain a synthetic product. During the reaction process, it was found that the scheme required harsh reaction conditions, and the yield and purity were unsatisfactory.

In another existing related technology, the acyl chloride derivative of theanine was condensed with tocopherol to obtain the desired synthetic product. However, it was also found that the reaction conditions remained harsh, and the yield and purity were similarly poor.

Therefore, there is an urgent need to develop a synthetic product based on tocopherol and theanine, together with a synthesis method that is efficient, energy-saving, and capable of reducing safety hazards and toxic or harmful issues in the production process.

### SUMMARY

The present invention aims to provide a synthetic product of tocopherol and theanine, namely a tocopherol derivative, and to overcome the disadvantages of conventional phenol-acid reactions, including low economic efficiency, harsh reaction conditions, and complicated operations. The invention provides a novel preparation method for tocopherol derivatives. The method is characterized by mild reaction conditions, simple operation, high yield, high efficiency, cost-effectiveness, and environmental friendliness, with promising application prospects.

Specifically, the present invention provides a tocopherol derivative characterized by the following structure:

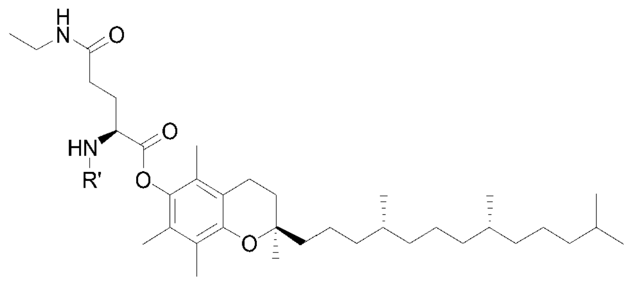

wherein R' is hydrogen or an acetyl group.

Preferably, R' is hydrogen. From the perspectives of product stability, usability, and diversification, one hydrogen atom of the $NH_2$ group may also be substituted with an acetyl group.

The tocopherol derivative can be used as an antioxidant and applied as an antioxidant additive in cosmetics, health foods, and pet products.

The present invention further provides a preparation method for the tocopherol derivative, characterized in that theanine and tocopherol are esterified in the presence of a catalyst and a co-catalyst to obtain the tocopherol derivative.

The catalyst is selected from compounds of the following structure:

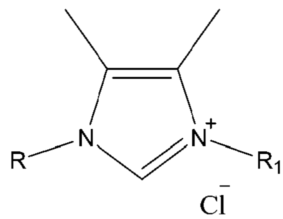

wherein R and $R_1$ are identical or different alkyl or aryl groups.

The specific reaction equation is shown as follows:

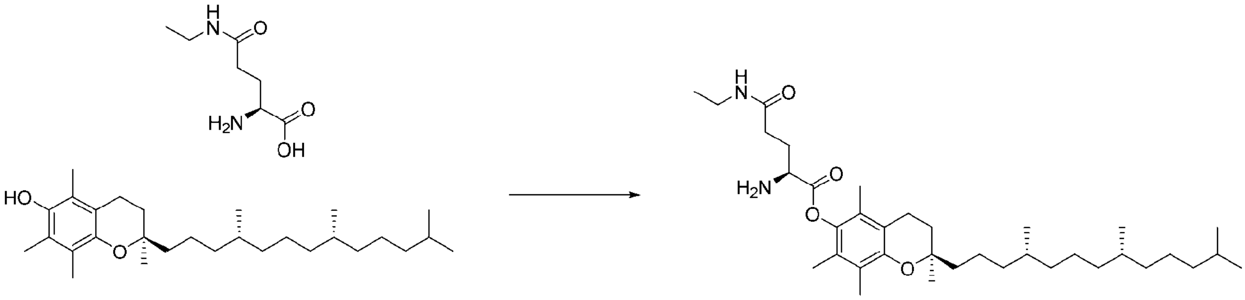

In the above catalyst, R and $R_1$ are preferably Me, n-Bu, or Ph. The most preferred catalysts are selected from the following compounds:

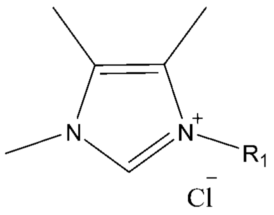

cat.1

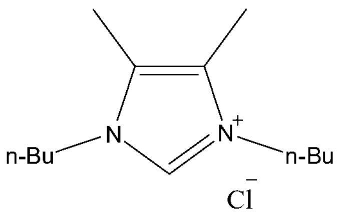

cat.2

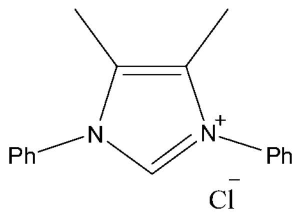

cat.3

The reaction mechanism of the catalyst in the preparation of the tocopherol derivative is as follows: the acidic hydrogen of the cationic portion of the catalyst serves as a hydrogen bond donor to activate the carbonyl group of theanine, while the oxygen anion of the anionic portion of the catalyst acts as a hydrogen bond acceptor to activate the phenolic hydroxyl group of tocopherol, thereby promoting the esterification reaction.

Taking catalyst cat.1 as an example, the activation mechanism of the reaction can be described as follows:

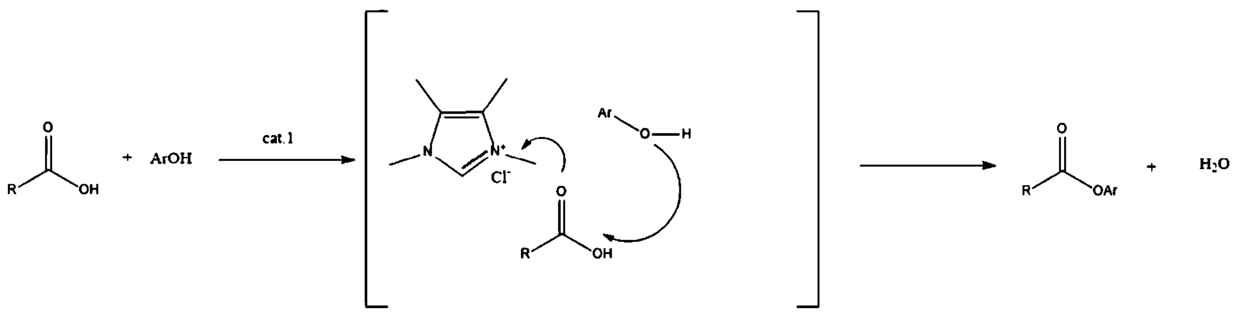


Furthermore, the preparation method of the tocopherol derivative according to the present invention is characterized in that: the molar amount of the catalyst added is 0.2-0.5% of the total molar amount of the reactants, preferably 0.2-0.4%, and most preferably 0.2-0.3%.

Further, the molar ratio of theanine to tocopherol is 1-1.5:1, preferably 1.2-1.5:1, and most preferably 1.4-1.5:1.

Further, the co-catalyst is sodium phosphate, which functions to activate the catalyst.

Further, the molar ratio of the co-catalyst to theanine is 0.1-0.5:1, preferably 0.1-0.3:1, and most preferably 0.1-0.2:1.

Further, the preparation method is characterized in that theanine, tocopherol, the catalyst, and the co-catalyst are mixed and reacted under stirring at room temperature for 4-6 hours, followed by post-treatment and purification to obtain the product.

Further, the reaction is carried out in a solvent. The solvent is selected from one or more of halogenated alkanes (e.g., dichloromethane, chloroform), ethers (e.g., tetrahydrofuran, dioxane, tert-butyl methyl ether, diethyl ether), nitriles (e.g., acetonitrile), amides (e.g., N,N-dimethylformamide), and sulfoxides (e.g., dimethyl sulfoxide).

Further, the method may additionally comprise adding molecular sieves in an amount of 0.1-1% of the total weight of the raw materials, preferably 0.1-0.5%, and most preferably 0.1-0.3%.

Further, the catalyst and the co-catalyst are used to catalyze the esterification reaction.
The present invention also proposes the use of the tocopherol derivative as an antioxidant.

## BRIEF DESCRIPTION OF THE DRAWINGS

Figure 1. illustrates the hydrogen spectrum of the tocopherol derivative provided in Example 1.
Figure 2.illustrates the reference curve for the scavenging effect of ascorbic acid (VC) on DPPH radicals.
Figure 3. illustrates the scavenging effect of the test product on DPPH radicals.
Figure 4. illustrates the scavenging effect of the test product on ·OH.

## DESCRIPTION OF THE EMBODIMENTS

Theanine, tocopherol, sodium phosphate, catalyst, and co-catalyst were mixed and stirred at room temperature for 4 hours. The reaction mixture was subjected to post-treatment and purification to afford the target product.
The structure of the product is represented as follows:

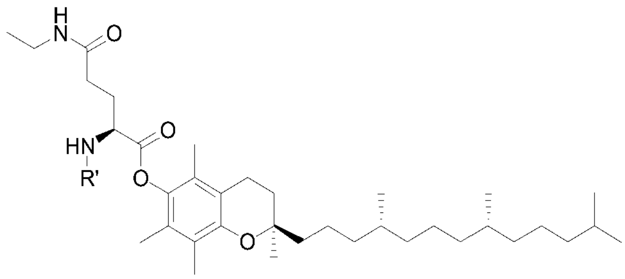

, R' = H; based on the requirements of derivative products, the obtained product may further react with an acetylation reagent (e.g., acetyl chloride) to yield a derivative in which R' is an acetyl group.
The catalyst is selected from The catalyst is selected from

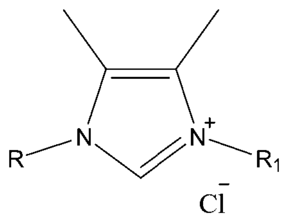
;

R and $R_1$ are identical or different alkyl or aryl groups. Examples of alkyl groups include straight-chain or branched alkyl groups containing no more than 10 carbon atoms, such as methyl, ethyl, propyl, isopropyl, butyl, isobutyl, pentyl, 2-methylbutyl, etc. Examples of aryl groups include phenyl, benzyl, p-tolyl, o-tolyl, m-tolyl, xylyl, p-methoxyphenyl, p-chlorophenyl, m-methoxyphenyl, m-chlorophenyl, o-methoxyphenyl, and o-chlorophenyl.
The preferred catalysts are selected from cat. 1, cat.2, and cat.3.

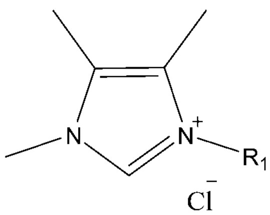
cat.1

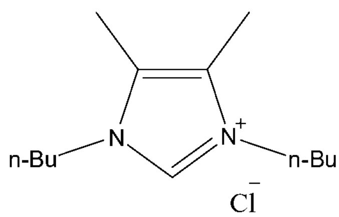
cat.2

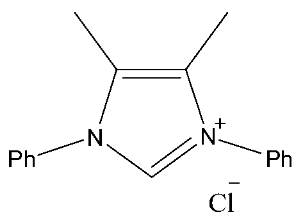
cat.3

The co-catalyst is sodium phosphate. The reaction solvent is selected from halogenated hydrocarbons (e.g.,

dichloromethane, chloroform), ethers (e.g., tetrahydrofuran, dioxane, tert-butyl methyl ether, diethyl ether), nitriles (e.g., acetonitrile), amides (e.g., N,N-dimethylformamide), or sulfoxides (e.g., dimethyl sulfoxide), either alone or in combination.

The molar amount of the catalyst is 0.2-0.5% of the total molar amount of the reactants, or 2-10% of the total weight. The molar ratio of theanine to tocopherol is selected within 1-1.5:1.The molar ratio of sodium phosphate to theanine is selected within 0.1-0.5:1.Additionally, 0.1-1% by weight of molecular sieves relative to the total raw materials may be added.

Sources of Raw Materials:

| Reagent | Supplier | Specification | Notes |
|---|---|---|---|
| Theanine | Shandong Pingju | 1 kg, 99% | Configuration: L-theanine |
| | Biotechnology Co., Ltd. | | |
| Tocopherol | Macklin | 1 kg, 97% | Configuration: DL-α-tocopherol |
| Catalyst cat.1 | Aladdin | 1 g, 99% | CAS: 141556-39-0 |
| Catalyst cat.2 | Aladdin | 1 g, 99% | CAS: 1020098-95-6 |
| Catalyst cat.3 | Aladdin | 1 g, 99% | CAS: 3052255-14-5 |
| Catalyst cat.4 | Aladdin | 1 g, 99% | CAS: 54016-70-5 |
| Catalyst cat.5 | Aladdin | 1 g, 99% | CAS: 2107416-41-9 |
| Sodium phosphate | Shanghai Haohong Biomedical Technology Co., Ltd. | 1 kg, 98% | |
| Tetrahydrofuran | Shanghai Haohong Biomedical Technology Co., Ltd. | AR | |
| Sodium bicarbonate | Shanghai Haohong Biomedical Technology Co., Ltd. | 1 kg, 98% | |
| Sodium chloride | Shanghai Haohong Biomedical Technology Co., Ltd. | 1 kg, 98% | |

Preferred Embodiments

Example 1. Preparation of theanine tocopherol ester catalyzed by cat.1

Into a 250 mL three-neck flask were added theanine (5 g), tocopherol (3.9 g), cat.1 (0.373 g), sodium phosphate (0.53 g), and tetrahydrofuran (100 mL). The mixture was stirred at room temperature for 4 h. The solid impurities were filtered off, 40 mL of water was added, and the mixture was stirred for 3 min. The organic phase was separated and dried to afford 5.9 g of pure theanine tocopherol ester (purity 97.6%), yield 88%. $^1H$ NMR (400 MHz, $CDCl_3$) δ 6.77 (t, J = 4.1 Hz, 1H), 3.92-3.81 (m, 1H), 3.41 (dd, J = 7.3, 6.6 Hz, 1H), 3.26-3.16 (m, 2H), 2.78 (dd, J = 7.8, 5.0 Hz, 1H), 2.72 (dd, J = 7.8, 5.0 Hz, 1H), 2.38-2.25 (m, 2H), 2.19-2.04 (m, 7H), 2.04-1.90 (m, 1H), 1.75 (dd, J = 7.8, 5.0 Hz, 1H), 1.73-1.19 (m, 25H), 1.13 (t, J = 6.4 Hz, 3H), 0.85 (dd, J = 6.8, 4.3 Hz, 9H), 0.79 (d, J = 6.7 Hz, 3H).LC-MS: 587.75 $[M+H]^+$.

Example 2: Preparation of Tocopherol-Theanine Ester Catalyzed by cat.2

Into a 250 mL three-neck flask were added theanine (5 g), tocopherol (3.9 g), cat.2 (0.363 g), sodium phosphate (0.53 g), and tetrahydrofuran (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 5.2 g (purity 94.3%), with a yield of 75%.

Example 3: Preparation of Tocopherol-Theanine Ester Catalyzed by cat.3

Into a 250 mL three-neck flask were added theanine (5 g), tocopherol (3.9 g), cat.3 (0.353 g), sodium phosphate (0.53 g), and tetrahydrofuran (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 6.2 g (purity 99.1%), with a yield of 92%.

Example 4: Preparation of Tocopherol-Theanine Ester Catalyzed by cat.3

Into a 250 mL three-neck flask were added theanine (5 g), tocopherol (3.9 g), cat.3 (0.353 g), sodium phosphate (0.53 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase, and drying to obtain pure tocopherol-theanine ester, 6.4 g (purity 99.3%), with a yield of 94%.

Example 5: Preparation of Tocopherol-Theanine Ester Catalyzed by cat.3 (Optimum Conditions)

Into a 250 mL three-neck flask were added theanine (5 g), tocopherol (3.9 g), cat.3 (0.353 g), sodium phosphate (0.53 g), molecular sieves (0.2 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase, then addition of 40 mL of saturated sodium chloride solution, and drying to obtain pure tocopherol-theanine ester, 6.9 g (purity 99.7%), with a yield of 98%.

Comparative Example 1

Into a 250 mL three-neck flask were added theanine (5 g), tocopherol (3.9 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase. Chromatographic analysis showed almost no product.

Comparative Example 2

Into a 250 mL three-neck flask were added theanine (5 g), tocopherol (3.9 g), cat.3 (0.35 g), and tetrahydrofuran (100 mL) as solvent. The mixture was stirred at room temperature for 4 hours. The solid impurities were filtered off. Chromatographic analysis showed almost no product.

Comparative Example 3Into a 250 mL three-neck flask were added theanine (5 g), tocopherol (3.9 g), cat.4

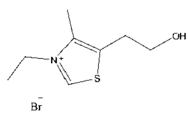

(0.35 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase. Chromatographic analysis showed almost no product.

Comparative Example 4

Into a 250 mL three-neck flask were added theanine (5 g), tocopherol (3.9 g), cat.5

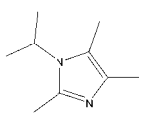

(0.35 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase. Chromatographic analysis showed almost no product.

Comparative Example 5Into a 250 mL three-neck flask were added theanine (5 g), tocopherol (3.9 g), boric acid

(0.3 g), oxalic acid (0.3 g), and tetrahydrofuran (100 mL) as solvent. The mixture was stirred at room temperature for 4 hours. The solid impurities were filtered off, followed by addition of 40 mL of saturated sodium bicarbonate solution, stirring for 3 minutes, separation of the organic phase. Chromatographic analysis showed almost no product.

Experimental Example 6

Into a 250 mL three-neck flask were added theanine (5 g), tocopherol (3.9 g), sodium phosphate (0.1 g), cat.3 (0.35 g), and tetrahydrofuran (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 4.1 g (purity 98.4%).

Experimental Example 7

Into a 250 mL three-neck flask were added theanine (5 g), tocopherol (3.9 g), sodium phosphate (0.9 g), cat.3 (0.35 g), and tetrahydrofuran (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 4.3 g (purity 98.1%).

Experimental Example 8

Into a 250 mL three-neck flask were added theanine (5 g), tocopherol (3.9 g), sodium phosphate (0.5 g), cat.3 (0.35 g), and ethanol (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 2.8 g (purity 91.1%).

Experimental Example 9

Into a 250 mL three-neck flask were added theanine (5 g), tocopherol (3.9 g), PBS (0.5 g), cat.3 (0.35 g), and tetrahydrofuran (100 mL) as solvent. The mixture was stirred at room temperature for 4 hours. The solid impurities were filtered off, followed by addition of 40 mL of water, stirring for 3 minutes, separation of the organic phase, and drying to obtain pure tocopherol-theanine ester, 3.3 g (purity 89.6%).

Experimental Example 10 Antioxidant Effect Test of Tocopherol-Theanine Ester (the test sample being the purified tocopherol-theanine ester synthesized in Example 1)

1. Determination of DPPH Radical Scavenging Capacity

1.1 Experimental Principle

The DPPH radical scavenging activity assay is an in vitro method to evaluate antioxidant activity. DPPH is a stable macromolecular free radical in organic solvents and exhibits a purple color in methanol or ethanol with maximum absorbance at 517 nm. The DPPH colorimetric method is based on the principle that a radical scavenger can donate an electron to pair with the unpaired electron of DPPH, thereby changing the purple color at 517 nm to yellow. The degree of absorbance change is linearly correlated with the extent of free radical scavenging, i.e., the stronger the scavenging ability of the radical scavenger, the lower the absorbance.

1.2 Experimental ReagentsDPPH (Sigma), PBS (Gibco), absolute ethanol (Sinopharm Reagent), petroleum ether (Sinopharm Reagent), vitamin C (CNW), absolute ethanol (Sinopharm Reagent).Main equipment: microplate reader (Tecan, Spark), micro-shaker (Qilinbeier, TS-92).

1.3 Experimental Methods

1.3.1 Construction of Reference Curve for DPPH Radical Scavenging by System Reference Substance

Ascorbic acid (VC) was used as the system reference substance and diluted with PBS to 12.5, 25, 50, 100, and 200 μg/mL at five gradient concentrations. The assay was conducted according to method 1.3.2, and results were calculated. A reference curve was plotted with the reference substance concentration as the x-axis and the DPPH radical scavenging rate as the y-axis.

1.3.2 In Vitro DPPH Radical Scavenging Assay

The test sample was prepared into solutions of corresponding concentrations. Reaction systems were formulated according to Table 1 with the specified reagent additions, mixed thoroughly, with five replicate wells for each concentration and one background control well.

Table 1: Reaction System of DPPH Radical Scavenging Assay

| Unit (μL) | 1 | 2 | 1 | 2 |
|---|---|---|---|---|
| DPPH ethanol solution | 180 | 0 | 180 | 0 |
| Test solution | 0 | 0 | 20 | 20 |
| Absolute ethanol | 0 | 180 | 0 | 180 |
| PBS | 20 | 20 | 0 | 0 |

The reaction systems were incubated at room temperature in the dark for 30 minutes. At the end of the reaction, absorbance (OD) values were measured at 517 nm, and the DPPH radical scavenging rate of the test sample was calculated using the following formula:

$$\text{Scavenging rate of test sample on DPPH radicals} = [(C1 - C2) - (T1 - T2)] / (C1 - C2) \times 100\%$$

Wherein:C1 - absorbance of blank system containing DPPH
C2 - absorbance of blank system without DPPH
T1 - absorbance of test sample group containing DPPH
T2 - absorbance of test sample group without DPPH

1.4 Results of In Vitro DPPH Radical Scavenging Assay

1.4.1 System Reference Curve of DPPH Radical Scavenging. See Table 2 and Figure 2.

Table 2: Analysis of System Reference Substance Results

| VC concentration (μg/mL) | 200 | 100 | 50 | 25 | 12.5 |
|---|---|---|---|---|---|
| DPPH radical scavenging rate (%) | 99.60 | 90.41 | 67.87 | 30.55 | 20.71 |
| | 99.65 | 90.43 | 72.65 | 33.04 | 21.33 |
| | 96.14 | 91.70 | 65.65 | 31.49 | 16.32 |
| | 100.61 | 96.01 | 64.59 | 34.01 | 22.89 |
| | 91.05 | 97.86 | 64.51 | 33.93 | 17.46 |

1.4.2 In Vitro DPPH Radical Scavenging Results of the Test Sample. See Table 3 and Figure 3.

Table 3: Analysis of DPPH Radical Scavenging Results of Test Sample

| Test sample concentration (% v/v) | DPPH radical scavenging rate (% v/v) | p | Significance |
|---|---|---|---|
| 0 | 0.31 ± 1.65 | / | / |
| 0.02 | 9.52 ± 1.34 | <0.001 | *** |
| 0.1 | 17.71 ± 1.40 | <0.001 | *** |
| 0.5 | 30.36 ± 1.44 | <0.001 | *** |
| 2 | 52.02 ± 1.32 | <0.001 | *** |

(continued)

| Test sample concentration (% v/v) | DPPH radical scavenging rate (% v/v) | p | Significance |
|---|---|---|---|
| 4 | 76.40 ± 4.73 | <0.001 | *** |
| Note: The number of asterisks indicates the level of statistical significance, where *** denotes high significance and * denotes low significance. | | | |

1.5 Conclusion

At concentrations of 0.02%-4%, the test sample significantly increased the DPPH radical scavenging rate compared with the control group ($p < 0.001$), demonstrating antioxidant activity.

2. Determination of Hydroxyl Radical (·OH) Scavenging Capacity

2.1 Experimental Principle

When the antioxidant function of the body declines or cell damage occurs, excessive free radicals are produced. Among reactive oxygen species, hydroxyl radical (·OH) has the highest reactivity ($10^7$-$10^{10}$ $M^{-1}$ $S^{-1}$) and is the most harmful to the body. Hydroxyl radicals are generated through the Fenton reaction: $H_2O_2 + Fe^{2+} \rightarrow \cdot OH + H_2O + Fe^{3+}$. The generated ·OH reacts with salicylic acid to produce a compound with specific absorbance at 520 nm. The addition of a test sample capable of scavenging ·OH reduces the amount of ·OH generated, thereby decreasing the amount of colored compound formed. The degree of absorbance change is related to the extent of ·OH scavenging; i.e., the stronger the scavenging ability, the lower the absorbance.

2.2 Experimental Methods

The test sample was prepared into solutions of corresponding concentrations. The reagents were added according to the reaction system in Table 4, and absorbance values were measured at 520 nm. The scavenging rate was calculated according to the following formula. The test sample was tested at five gradient concentrations, with each concentration measured in five replicates.

$$\text{Scavenging rate of test sample on } \cdot OH \text{ radicals} = [\,(A - C) - (B - D)\,] / (A - C) \times 100\%$$

Table 4 Reaction System of Hydroxyl Radical Scavenging Assay

| A (Control) | B (Test sample) | C (Control reference) | C (Sample reference) |
|---|---|---|---|
| 0.2 mL distilled water | 0.2 mL test solution | 0.2 mL distilled water | 0.2 mL test solution |
| 0.2 mL $FeSO_4$ | 0.2 mL $FeSO_4$ | 0.2 mL$FeSO_4$ | 0.2 mL $FeSO_4$ |
| 0.4 mL $H_2O_2$ | 0.4 mL $H_2O_2$ | 0.4 mL $H_2O_2$ | 0.4 mL $H_2O_2$ |
| Reaction 10 min | Reaction 10 min | Reaction 10 min | Reaction 10 min |
| 0.2 mL salicylic acid ethanol solution | 0.2 mL salicylic acid ethanol solution | 0.2 mL absolute ethanol solution | 0.2 mL absolute ethanol solution |

2.3 Results of In Vitro Hydroxyl Radical Scavenging Test See Table 5.

Table 5 Analysis of Hydroxyl Radical Scavenging Results of Test Sample

| Test sample concentration (% v/v) | ·OH scavenging rate (% v/v) | p | Significance |
|---|---|---|---|
| 0 | 0.11 ± 1.65 | / | / |
| 0.02 | 9.30 ± 1.34 | <0.001 | *** |
| 0.1 | 22.98 ± 1.40 | <0.001 | *** |

(continued)

| Test sample concentration (% v/v) | ·OH scavenging rate (% v/v) | p | Significance |
|---|---|---|---|
| 0.5 | 29.00 ± 1.44 | <0.001 | *** |
| 2 | 42.50 ± 1.32 | <0.001 | *** |
| 4 | 54.10 ± 4.73 | <0.001 | *** |
| Note: The number of asterisks indicates the level of statistical significance, where *** denotes high significance and * denotes low significance. | | | |

2.4 Experimental Conclusion

At concentrations of 0.02%-4%, the test sample significantly increased hydroxyl radical scavenging rates compared with the control group ($p < 0.001$), demonstrating antioxidant activity.

## Claims

1. A tocopherol derivative, **characterized in that**, the tocopherol derivative is a compound shown in following structure:

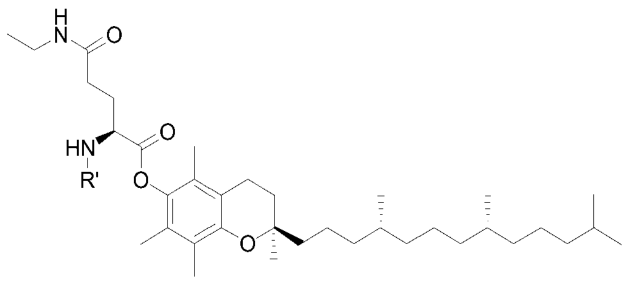

wherein R' is hydrogen or an acetyl group.

2. A method for preparing a tocopherol derivative of claim 1, **characterized in that**, a theanine and a tocopherol are esterified in presence of a catalyst and a co-catalyst to obtain the tocopherol derivative;

   the catalyst is selected from compounds shown in following structure:

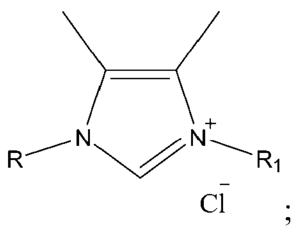

;

   R and $R_1$ are identical or different alkyl or aryl groups;
   the alkyl groups include straight-chained or branched alkyl groups containing no more than 10 carbon atoms; the aryl groups selected from phenyl, benzyl, p-tolyl, o-tolyl, m-tolyl, xylyl, p-methoxyphenyl, p-chlorophenyl, m-methoxyphenyl, m-chlorophenyl, o-methoxyphenyl, and o-chlorophenyl.

3. The method of claim 2, **characterized in that**, a molar amount of the catalyst added is 0.2-0.5% of a total molar amount of reactants.

4. The method of claim 2, **characterized in that**, a molar ratio of the theanine to the tocopherol is 1-1.5:1.

5. The method of claim 2, **characterized in that**, the co-catalyst is sodium phosphate.

6. The method of claim 2, **characterized in that**, a molar ratio of the co-catalyst to the theanine is 0.1-0.5:1.

7. The method of claim 2, **characterized in that**, the theanine, the tocopherol, the catalyst, and the co-catalyst are mixed and reacted under stirring at room temperature for 4-6 hours, followed by post-treatment and purification to obtain products.

8. The method of claim 7, **characterized in that**, sieves are added.

9. Use of the tocopherol derivative of claim 1 and the tocopherol derivative prepared by the method of any one of claims 2 to 8 as an antioxidant.

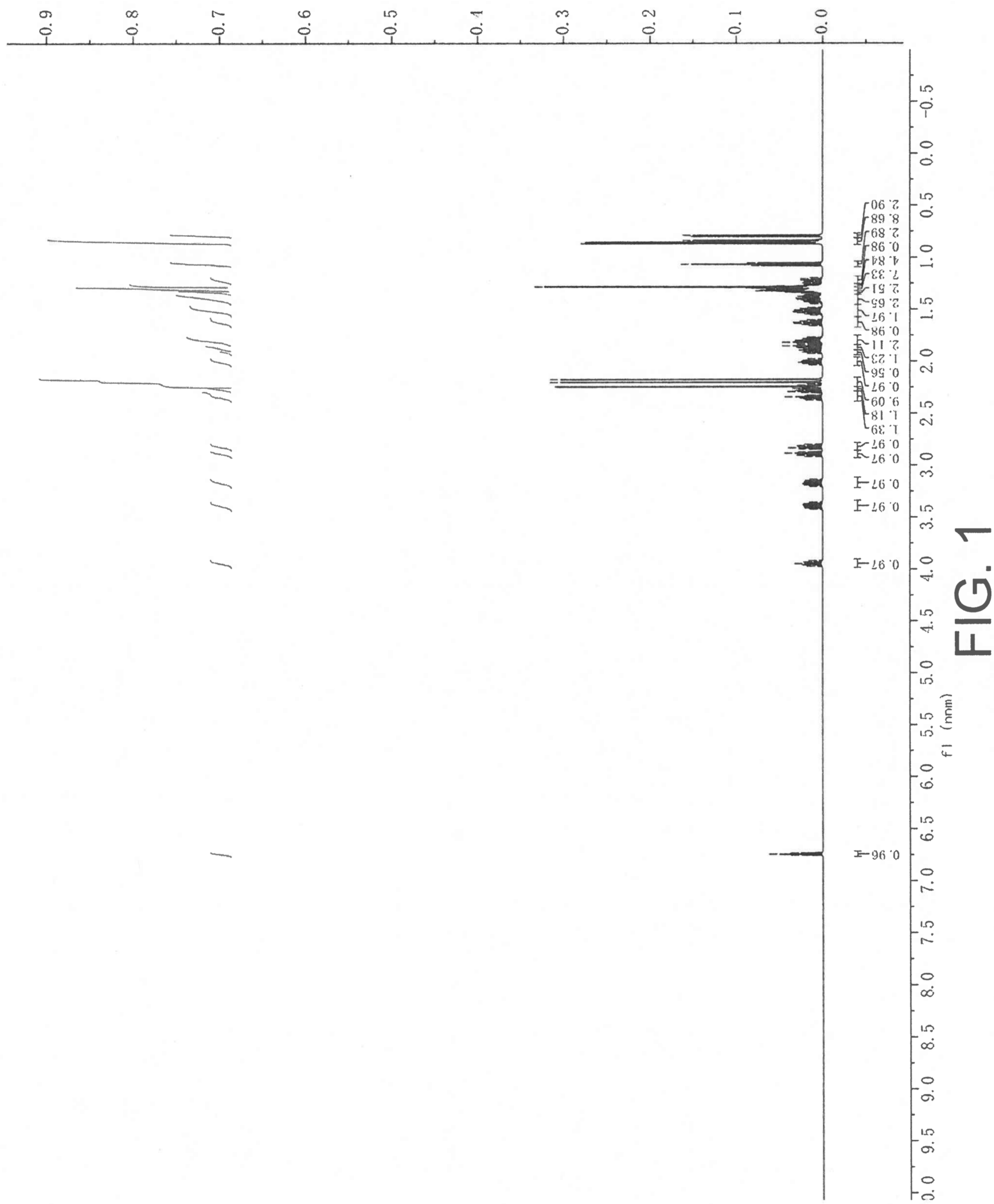


FIG. 1

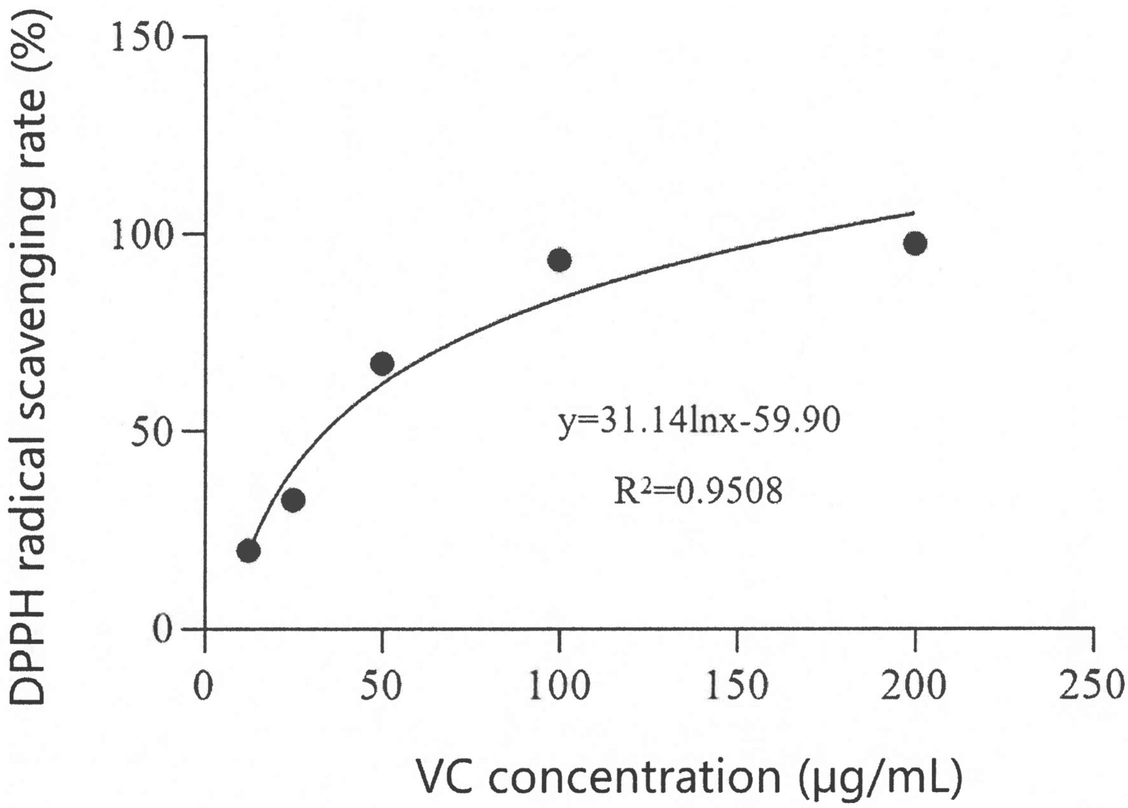
DPPH radical scavenging rate (%)
150
100
50
0
0
50
100
150
200
250
VC concentration (μg/mL)
y=31.14lnx-59.90
R²=0.9508

FIG. 2

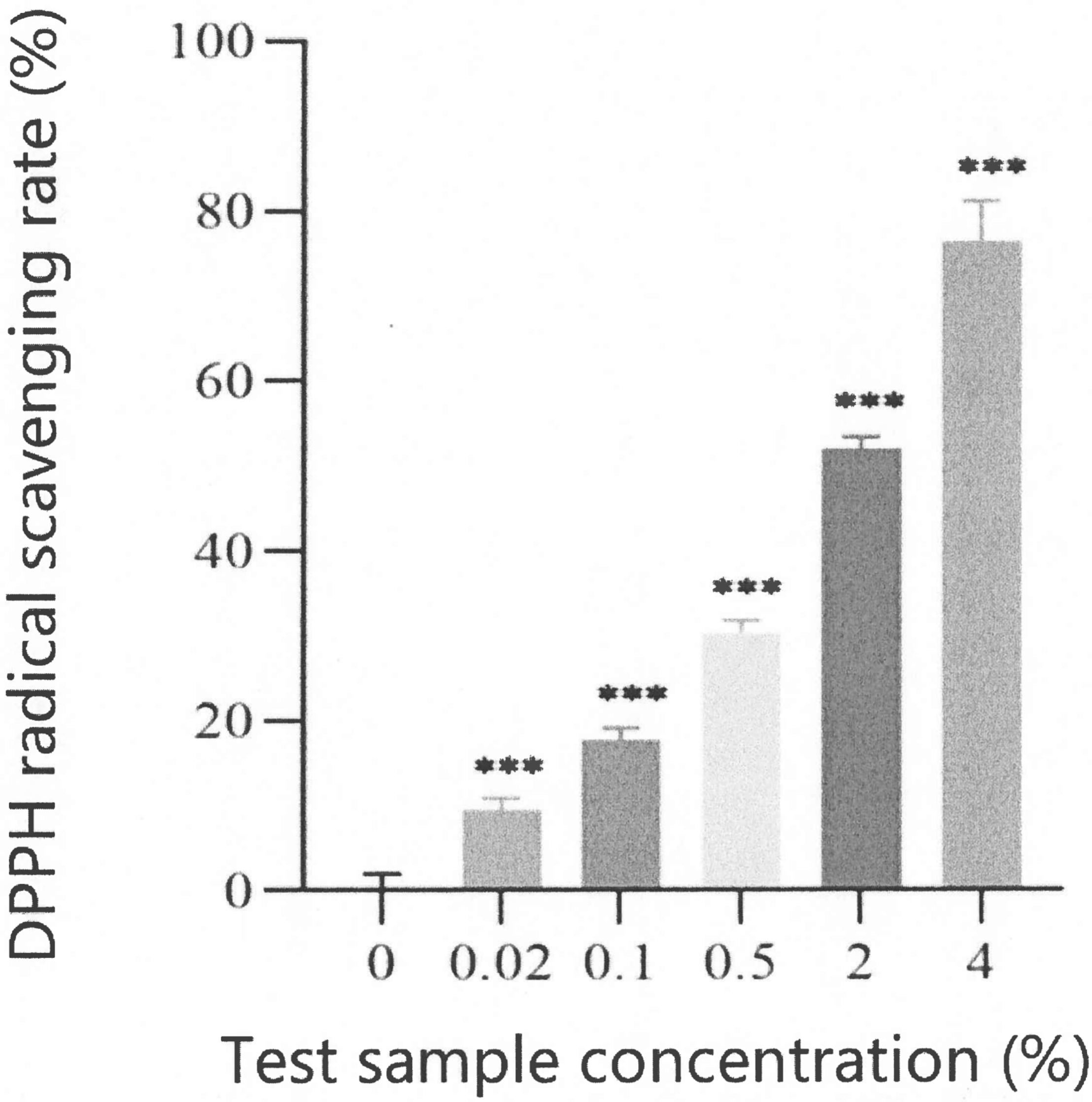
DPPH radical scavenging rate (%)
100
80
60
40
20
0
***
***
***
***
***
0
0.02
0.1
0.5
2
4
Test sample concentration (%)

FIG. 3

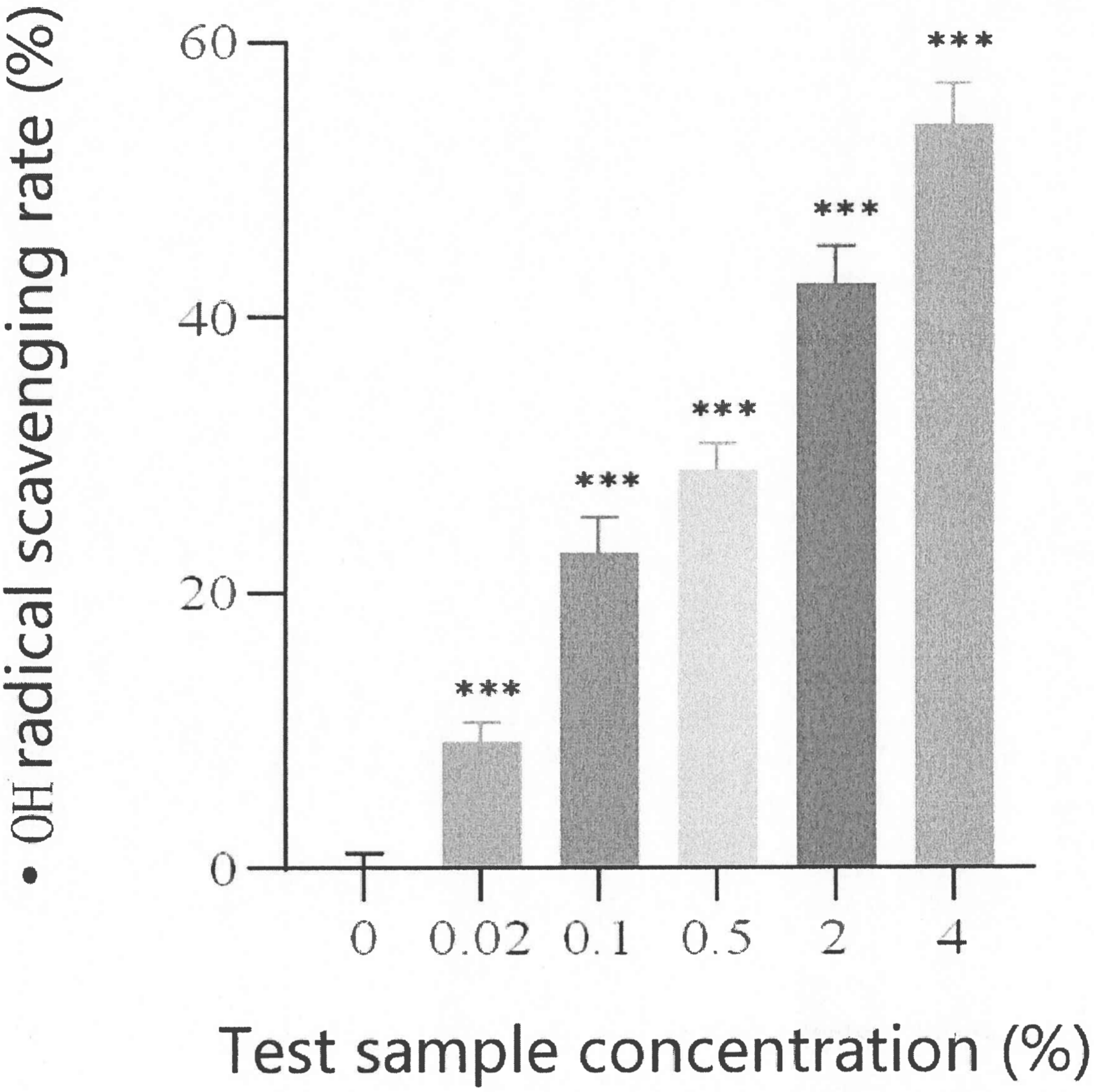
• OH radical scavenging rate (%)
60
40
20
0
***
***
***
***
***
0
0.02
0.1
0.5
2
4
Test sample concentration (%)

FIG. 4

Europäisches Patentamt
European Patent Office
Office européen des brevets

# EUROPEAN SEARCH REPORT

Application Number
EP 25 21 3183

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARRA FABIO ET AL: "Synthesis, hydrolysis, and skin retention of amino acid esters of [alpha]-tocopherol", JOURNAL OF PHARMACEUTICAL SCIENCES, vol. 98, no. 7, 7 July 2009 (2009-07-07), pages 2364-2376, XP093396987, Retrieved from the Internet: URL:https://doi.org/10.1002/jps.21608> | 1,9 | INV. C07D311/72 A61K8/67 |
| A | * abstract; figures; compound 10 * ----- | 2-8 | |
| A | WO 94/13624 A1 (KYOWA HAKKO KOGYO KK [JP]; KAMIYA TOSHIKAZU [JP] ET AL.) 23 June 1994 (1994-06-23) * page 6; compound 1 * ----- | 1-9 | |
| A | GAGIC ZARKO ET AL: "Synthesis of the vitamin E amino acid esters with an enhanced anticancer activity and in silico screening for new antineoplastic drugs", EUROPEAN JOURNAL OF PHARMACEUTICAL SCIENCES- AUTHOR MANUSCRIPT, vol. 88, 1 June 2016 (2016-06-01), pages 59-69, XP093397013, NL ISSN: 0928-0987, DOI: 10.1016/j.ejps.2016.04.008 Retrieved from the Internet: URL:https://pdf.sciencedirectassets.com/271051/1-s2.0-S0928098716X00061/1-s2.0-S0928098716301099/main.pdf?hash=e5e5c2b6ab2e75191ebfaa6168b722827a390e06608e7a6bdda99249b6ccd1bf&host=68042c943591013ac2b2430a89b270f6af2c76d8dfd086a07176afe7c76c2c61&pii=S0928098716301099&tid=spdf-43c07263-fbc6-47ce-9c1f-2e6> * abstract; compound 4c * ----- | 1-9 | TECHNICAL FIELDS SEARCHED (IPC) C07D A61K |

The present search report has been drawn up for all claims

| Place of search | Date of completion of the search | Examiner |
|---|---|---|
| Munich | 18 May 2026 | Miniejew, Catherine |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT ON EUROPEAN PATENT APPLICATION NO. EP 25 21 3183

This annex lists the patent family members relating to the patent documents cited in the above-mentioned European search report. The members are as contained in the European Patent Office EDP file on
The European Patent Office is in no way liable for these particulars which are merely given for the purpose of information.

18-05-2026

| Patent document cited in search report | Publication date | Patent family member(s) | Publication date |
|---|---|---|---|
| WO 9413624 A1 | 23-06-1994 | AT E159713 T1 | 15-11-1997 |
| | | CN 1095725 A | 30-11-1994 |
| | | DE 69314942 T2 | 30-04-1998 |
| | | EP 0627410 A1 | 07-12-1994 |
| | | ES 2110213 T3 | 01-02-1998 |
| | | HK 1000769 A1 | 24-04-1998 |
| | | JP 3424930 B2 | 07-07-2003 |
| | | KR 950700236 A | 16-01-1995 |
| | | TW 257750 B | 21-09-1995 |
| | | US 5468474 A | 21-11-1995 |
| | | WO 9413624 A1 | 23-06-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 141556-39-0
- *CHEMICAL ABSTRACTS*, 1020098-95-6
- *CHEMICAL ABSTRACTS*, 3052255-14-5
- *CHEMICAL ABSTRACTS*, 54016-70-5
- *CHEMICAL ABSTRACTS*, 2107416-41-9